# EUROPEAN PATENT APPLICATION

(11) **EP 0 770 683 A1**
(43) Date of publication of application: **02.05.1997**
(21) Application number: 96115557.9
(22) Date of filing: 27.09.1996
(51) Int. Cl.: C12P 7/18

(54) **Method for producing erythritol**

(30) Priority: 04.10.1995 JP 257664/95
(71) Applicant: Mitsubishi Chemical Corporation, Chiyoda-ku, Tokyo (JP)
(72) Inventor: Ueda, Makoto, Mitsubishi Chem. Corp., Yokohama-shi, Kanagawa (JP); Yamagishi, Kenji, Mitsubishi Chem. Corp., Yokohama-shi, Kanagawa (JP)
(74) Representative: Hansen, Bernd, Dr. Dipl.-Chem.

(57) **Abstract**

Provided is a method for producing erythritol, comprising the steps of: cultivating yeast which belongs to the genus Yallowia and which has an ability to produce erythritol from a fermentable saccharide in a medium containing a fermentable saccharide as a main carbon source; and collecting erythritol from the culture.

According to the method of the present invention, erythritol can be produced from an inexpensive fermentable saccharide such as glucose and the like, at a high yield and a good efficiency.

## Description

### Field of the Invention

The present invention relates to a method for producing erythritol. More specifically, the present invention relates to an industrially advantageous method for producing erythritol through fermentation using yeast.

### Background of the Invention

As a method for producing erythritol, a method in which yeast belonging to the genus Trigonopsis or Candida is cultivated in a medium containing glycerol as a carbon source ( Japanese Patent Publication No. 47-41549 ) and a method in which yeast belonging to the genus Candida, Torulopsis or Hansenula is cultivated in a medium containing hydrocarbons as a carbon source ( Japanese Patent Publication No. 51-21072 ) are known. However, these mehtods have not yet been industrialized because the starting material used as the carbon source is not appropriate in the actual industrial production.

Also known is a method in which Moniliella tomentosa var. pollinis is cultivated in a medium containing saccharides such as glucose and the like as a carbon source for producing erythritol ( Japanese Patent Publication No. 60-110295 ). This method is excellent in that glucose which is an inexpensive, safe starting material is used and an amount of erythritol produced is large. However, foaming occurs drastically during the cultivation, and an ordinary antifoam reagent is not useful for controlling the foaming. Therefore, a large amount of expensive xanthan gum has to be added. Accordingly, this method is not necessarily said to be advantageous in the industrial production.

### Summary of the Invention

It is an object of the present invention to provide a method for producing erythritol from an inexpensive starting material which can easily be supplied, at a high yield and a low cost.

The present inventors have conducted studies with respect to a method for producing erythritol from an inexpensive starting material, and have consequently found that yeast belonging to the genus Yallowia produces a large amount of erythritol from fermentable saccharides such as glucose, fructose and the like. This finding has led to the completion of the present invention.

That is, the present invention is to provide a method for producing erythritol, comprising the steps of: cultivating yeast which belongs to the genus Yallowia and which has an ability to produce erythritol from a fermentable saccharide in a medium containing a fermentable saccharide as a main carbon source; and collecting erythritol from the culture.

According to preferred embodiments of the present invention, there are provided the above-mentioned method wherein the yeast belonging to the genus Yallowia is Yarrowia lipolytica, the above-mentioned method wherein the fermentable saccharide is selected from the group consisting of glucose, fructose and glycerol, the above-mentioned method wherein the concentration of the fermentable saccharide in the medium is between 20 and 50%, and the above-mentioned method wherein the cultivation temperature is between 25°C and 35°C.

In accordance with the method of the present invention, erythritol can be produced from an inexpensive fermentable saccharide such as glucose and the like, at a high yield and a good efficiency.

### Detailed Description of the Invention

The present invention will be described in detail below.

The yeast which is used in the present invention may be any yeast which belongs to the genus Yallowia and which has an ability to produce erythritol from fermentable saccharides. Further, the yeast may be any type of strains including, for example, mutant strains obtained by irradiation of ultraviolet rays (UV) or treatment with N-methl-N-nitrosoguanidine (NTG), ethylmethanesulfonate (EMS), nitrous acid or acridine, etc. and recombinant strains derived by genetic techniques such as cell fusion and genetic recombination methods. As the yeast which belongs to the genus Yallowia and which has the above-mentioned ability, Yallowia lipolytica is preferable. Yallowia lipolytica is known yeast. Various strains thereof have been deposited in the depositary authorities, and therefore, these can easily be obtained. A specific example thereof is Yallowia lipolytica ATCC 8661 which is the strain deposited in American Type Culture Collection (ATCC, 12301 Parklawn Drive , Rockville, Maryland 20852, U. S. A.). This strain can be obtained from the above-mentioned depositary authority.

Examples of the main carbon source of the medium which is used to cultivate the yeast include fermentable saccharides such as glucose, fructose, glycerol, etc. These fermentable saccharides can be used either singly or in combination. The concentration of the fermentable saccharide(s) is not particularly limited. It is advantageous that the concentration is as high as possible unless the formation of erythritol is inhibited. A preferable concentration of that is between 20 and 50% (w/v).

The nitrogen source includes organic or inorganic nitrogen compounds such as ammonium salt, urea, peptone, yeast extract and corn steep liquor. As inorganic salts; phosphate, sulfate, salts of metals such as magnesium, potassium, manganese, iron and zinc can be used. Factors that expedite the growth of the yeast may be added as required. Examples thereof include vitamins, nucleotides and amino acids. It is advisable to add an appropriate amount of a commercial antifoam reagent to restrain from foaming during the cultivation.

The medium which is used at the outset of the fermentation is adjusted to a pH of from 3 to 7, preferably from 3 to 4.5. The temperature is adjusted to from 25°C to 35°C, preferably from 27°C to 32°C during the cultivation.

In the cultivation, cells may be inoculated in the medium directly from a slant cultivation. However, it is preferable to inoculate a culture solution which is obtained by cultivating cells in a liquid medium for from 1 to 4 days. The cultivation is conducted under an aerobic condition with aeration or shaking. It is preferable to conduct the cultivation until the main carbon source is completely consumed. The cultivation time is usually between 3 and 8 days. The amount of erythritol produced in the culture solution can be measured through gas chromatography or high-performance liquid chromatography.

Erythritol which is accumulated in the culture solution in this manner is separated from the culture and purified in a usual manner. Specifically, after the solids are removed through centrifugation, filtration, and so on, the residue is decolored and desalted using an activated carbon or an ion exchange resin, and the thus-treated solution is crystallized, whereby erythritol can be separated and purified.

### Description of the Preferred Embodiments

The present invention is illustrated more specifically by referring to the following Examples. However, the present invention is not limited thereto unless it deviates from the scope of the present invention.

### Example 1

50 ml of a medium containing 20% (w/v) of glucose and 1% of yeast extract were charged into a cotton-plugged 500-ml Erlenmeyer flask, and were sterilized at 120°C for 20 minutes. Yallowia lipolytica ATCC 8661 strain was inoculated in the thus-treated medium, and was cultivated at 27°C for 7 days while being shaken. The concentration of erythritol in the culture solution was measured by using high-performance liquid chromatography. As a result, it was found to be 92 g/liter. The yield based on glucose was 46%. The culture solution was centrifuged, and the solids were separated through filtration. The residue was decolored with an activated carbon. The clear solution was desalted using a strongly basic cation exchange resin (Diaion SKIB H-type) and a weakly basic anion exchange resin (Diaion WA30 OH-type). The resulting solution was concentrated at 50°C under reduced pressure until the concentration of erythritol reached 60%, and the concentrate was allowed to stand in a dark position to obtain crystals which had a melting point of 121°C and which had a sweet taste. The molecular formula thereof was C₄H₁₀O₄. The retention time of the liquid chromatography and the IR spectrum agreed with those of a standard product. Thus, the above-obtained product was identified as erythritol.

### Example 2

50 ml of a medium containing 1.0% of yeast extract and 20% of glucose were charged into a cotton-plugged 500-ml Erlenmeyer flask, and were sterilized at 120°C for 20 minutes. Yallowia lipolytica ATCC 8661 strain was inoculated in the thus-treated medium, and was cultivated at 27°C for 4 days while being shaken. This culture was inoculated in the above-mentioned medium at a ratio of 10%, and was cultivated in the above-mentioned manner. Furthermore, the resulting culture solution was inoculated in a medium containing glucose in the following concentration, and was cultivated. That is, a medium containing 1.0% of yeast extract and from 20 to 60% of glucose was charged into a 500-ml Erlenmeyer flask in an amount of 50 ml, and was inoculated and cultivated for 7 days while being shaken as mentioned above. After the completion of the cultivation, the concentration of erythritol in the culture solution was measured by using high-performance liquid chromatography. Consequently, the amount of erythritol produced in each concentration of glucose in the medium was as follows.

| Concentration of glucose in the medium (%) | Amount of erythritol produced (g/liter) |
|---|---|
| 20 | 72.6 |
| 30 | 98.6 |
| 40 | 73.6 |
| 50 | 57.2 |
| 60 | 3.7 |

### Example 3

50 ml of a medium containing 20% (w/v) of fructose and 1% of yeast extract were charged into a cotton-plugged 500-ml Erlenmeyer flask, and were sterilized at 120°C for 20 minutes. Yallowia lipolytica ATCC 8661 strain was inoculated in the thus-treated medium, and was cultivated at 27°C for 7 days while being shaken. The concentration of erythritol in the culture solution was measured by using high-performance liquid chromatography. Consequently, it was found to be 53.4 g/liter.

### Example 4

50 ml of a medium containing 20% (w/v) of glycerol and 1% of yeast extract were charged into a cotton-plugged 500-ml Erlenmeyer flask, and were sterilized at 120°C for 20 minutes. Yallowia lipolytica ATCC 8661 strain was inoculated in the thus-treated medium, and was cultivated at 27°C for 7 days while being shaken. The concentration of erythritol in the culture solution was measured by using high-performance liquid chromatography. Consequently, it was found to be 43.2 g/liter.

### Example 5

50 ml of a medium containing 30% of glucose and 1% of yeast extract were charged into each of six cotton-plugged 500-ml Erlenmeyer flasks, and were sterilized at 120°C for 20 minutes. Yallowia lipolytica ATCC 8661 strain was inoculated in the thus-treated medium, and was cultivated at 27°C for 4 days while being shaken. 17 liters of a medium containing 30% of glucose and 1% of yeast extract were charged into a 30-liter cultivation tank, and 500 ppm of an antifoam reagent CA330 ( made by Nippon Oils and Fats Co., Ltd. ) were added thereto. The mixture was sterilized at 120°C for 20 minutes. The above-mentioned culture was inoculated in the thus-sterilized medium, and was cultivated at 27° C for 4 days. 17 liters of a medium which contained 40% (w/v) of glucose, 2% of yeast extract and 0.1% of thiamine hydrochloride and which was adjusted to a pH of 4.0 were charged into a 30-liter cultivation tank. An antifoam reagent CA330 ( 500 ppm ) was added thereto, and the mixture was sterilized at 120°C for 20 minutes. The above-obtained culture solution (1.7 liters) was aseptically taken, inoculated in the above-sterilized medium, and cultivated at 27°C. After 96 hours of the cultivation, glucose was completely consumed. At that time, the concentration of erythritol was 205.5 g/liter. Furter, there was no conspicuous foaming during the cultivation.

### Example 6

50 ml of a medium containing 1.0% of yeast extract and 20% of glucose were charged into a cotton-plugged 500-ml Erlenmeyer flask, and were sterilized at 120°C for 20 minutes. Yallowia lipolytica ATCC 8661 strain was inoculated in the thus-treated medium, and was cultivated at 27°C for 4 days while being shaken. This culture was inoculated in the above-mentioned medium at a ratio of 10%, and was cultivated in the above-mentioned manner. Furthermore, the resulting culture solution was inoculated in a medium containing glucose in the following concentration, and was cultivated. That is, a medium containing 1.0% of yeast extract and from 10 to 60% of glucose was charged into a 500-ml Erlenmeyer flask in an amount of 50 ml, and was inoculated and cultivated for 7 days while being shaken as mentioned above. After the completion of the cultivation, the concentration of erythritol in the culture solution was measured by using high-performance liquid chromatography. Consequently, the amount of erythritol produced in each concentration of glucose in the medium was as follows.

| Concentration of glucose in the medium (%) | Amount of erythritol produced (g/liter) |
|---|---|
| 10 | 0.0 |
| 20 | 60.6 |
| 30 | 77.9 |
| 40 | 92.7 |
| 50 | 37.2 |
| 60 | 2.3 |

### Example 7

50 ml of a medium containing 1.0% of yeast extract and 20% of glucose were charged into a cotton-plugged 500-ml Erlenmeyer flask, and were sterilized at 120°C for 20 minutes. Yallowia lipolytica ATCC 8661 strain was inoculated in the thus-treated medium, and was cultivated at 27°C for 4 days while being shaken. 500 ml of a medium containing 1.0% of yeast extract, 40% of glucose and 500 ppm of an antifoam reagent CA330 were charged into a 1-liter cultivation tank were sterilized at 120°C for 20 minutes. The above-mentioned culture was inoculated in the thus-sterilized medium, and was cultivated for 96 hours respectively at each temperature which was selected between 20°C to 37°C as the following. After the completion of the cultivation, the concentration of erythritol in the culture solution was measured by using high-performance liquid chromatography. Consequently, the amount of erythritol produced in each cultivation temperature was as follows.

| Cultivation temperature(°C) | Amount of erythritol produced (g/liter) |
|---|---|
| 20 | 30.0 |
| 25 | 61.4 |
| 30 | 75.4 |
| 32 | 88.8 |
| 35 | 47.4 |
| 37 | 0.0 |

## Claims

1. A method for producing erythritol, comprising the steps of:
cultivating yeast which belongs to the genus Yallowia and which has an ability to produce erythritol from a fermentable saccharide in a medium containing a fermentable saccharide as a main carbon source; and
collecting erythritol from the culture.

2. The method of claim 1, wherein the yeast belonging to the genus Yallowia is Yarrowia lipolytica.

3. The method of claim 1, wherein the fermentable saccharide is selected from the group consisting of glucose, fructose and glycerol.

4. The method of claim 1, wherein the concentration of the fermentable saccharide in the medium is between 20 and 50%.

5. The method of claim 1, wherein the cultivation temperature is between 25°C and 35°C.
